# EUROPEAN PATENT SPECIFICATION

(11) **EP 3 383 270 B1**
(45) Date of publication and mention of the grant of the patent: **27.08.2025**
(21) Application number: 16805425.2
(22) Date of filing: 02.12.2016
(51) Int. Cl.: A61B 5/157, B01L 3/00, G01N 1/02, G01P 13/00

(54) **LIQUID SAMPLE COLLECTION APPARATUS**
VORRICHTUNG ZUR ENTNAHME FLÜSSIGER PROBEN
APPAREIL DE COLLECTE D'ÉCHANTILLON DE LIQUIDE

(30) Priority: 03.12.2015 GB 201521390
(43) Date of publication of application: 10.10.2018
(73) Proprietor: University Of Ulster, County Londonderry BT52 1SA (GB)
(72) Inventor: GIBSON, David, Londonderry County Londonderry BT47 2JQ (GB); MCLAUGHLIN, James, Belfast County Antrim BT7 2GH (GB); MAGEE, Justin, Dungiven County Londonderry BT47 4JZ (GB)
(74) Representative: FRKelly
(86) International application number: PCT/EP2016/079581
(87) International publication number: WO 2017/093480

(56) References cited:
- EP-A1- 2 275 034
- EP-A1- 2 275 034
- EP-A2- 0 780 679
- EP-B1- 0 780 679
- WO-A1-2007/073392
- WO-A1-2013/000357
- WO-A1-2013/000357
- WO-A1-2014/058750
- WO-A1-2014/058750
- WO-A1-99/44507
- GB-A- 2 436 616
- GB-A- 2 463 914
- US-A- 4 995 402
- US-A- 4 995 402
- US-A1- 2004 242 982
- US-A1- 2004 242 982
- US-A1- 2009 011 518
- US-A1- 2009 011 518

## Description

### Field of the Invention

This invention relates to a liquid sample collection apparatus and in particular to a liquid sample collection apparatus for the collection of blood samples.

### Background to the Invention

In patient populations with chronic conditions living some distance from hospitals, collection of a series of blood samples to monitor disease activity in a timely fashion is difficult. The ability to monitor markers of inflammation and gauge a patient's response to treatments for example in arthritis patients, is not just important in early stage disease but also in patients with established disease. Patients with chronic arthritis tend to lose self-confidence in managing their condition and a substantial proportion can suffer recurrent disease flare- ups. These flare-ups are costly in terms of appointments with GP's and specialist clinics and result in significant work disability.

Being able to reliably define and report a disease 'flare' in arthritis is currently problematic as there are no objective measures available to the patient while at home. The clear definition of a flare would help justify an increase in immunosuppressant dose or a course of steroids. Currently patients make a visit to their local GP or hospital clinic, at 6 week intervals at best. In reality this means that opportunities to sample during an active 'flare' are often missed and an informed intervention is not possible. Home monitoring is constrained by the costs of making phlebotomists available to make frequent home visits to patients.

Also for markers which are unstable at ambient temperatures, blood samples need to be refrigerated and rapidly transported to the analysis laboratory. Therefore there exists the need to provide the ability for the patient to collect their own blood samples in their own home and send these at ambient temperature to a laboratory would enable 'remote' monitoring of chronic disease.

EP2275034A1 discloses an apparatus and method for blood sample acquisition and testing. The apparatus incorporates a test strip (10) having a blood sample volume detection area (12) in which a blood sample is accumulated and a minimum blood sample volume is detected. Once a minimum blood sample volume is detected, the test strip is moved with respect to the blood sample extraction site so that a bending surface of the test strip is presented to the blood sample, utilizing the surface tension of the blood sample droplet to facilitate movement of the blood sample to a measurement site on the strip where blood glucose in the sample is measured.

US4995402A discloses assays for determining an analyte, and sensing means used in conjunction with such assays. More particularly, this invention relates to assays for analytes which employ as a tracer in the assays a ligand labeled with mercury or labeled with mercury releasing means and to an assay kit containing this tracer. The present invention also relates generally to the field of quantitative analysis of a desired constituent within a liquid and more particularly to a novel system by which one or more constituents within or forming part of a whole blood specimen from a medical patient or an animal or like liquid sample may be quantitatively ascertained inexpensively, for example, using a finger prick droplet blood specimen, at a site selected by the user, including the immediate vicinity of the patient, at the time shortly after the liquid specimen is obtained.

### Summary of the Invention

The present disclosure provides a liquid sample collection apparatus as detailed in claim 1. Advantageous features are provided in dependent claims.

Accordingly the present invention provides a liquid sample collection apparatus comprising, a port for receiving a liquid sample, a collector for storing said liquid sample, at least one conduit coupling said port to said collector, and a sample detection apparatus comprising a sensor for detecting said liquid in said collector and generating an output depending on the quantity of said liquid detected in said collector, and a controller responsive to said sensor output and configured to determine that said sample has been collected when said sensor output indicates that a quantity of said liquid above a threshold amount is detected in said collector.

Ideally, the sensor comprises first and second spaced apart electrodes coupled to said collector, said sensor output depending on the electrical conductance of said collector between said first and second electrodes, said electrical conductance depending on the quantity of liquid collected in use by said collector.

Preferably, the sensor comprises an electrical circuit in which said first and second electrodes serve as a switch, said switch being open or closed depending on the electrical conductance of said collector between said first and second electrodes.

Ideally, the first and second electrodes are mechanically connected to each other by said collector and are selectively electrically connected by said collector depending on the electrical conductance of said collector between said first and second electrodes.

Preferably, the first and second electrodes are configured to be electrically connected to each other when a quantity of said liquid above a threshold amount is collected by said collector.

Ideally, one of said electrodes is located adjacent an inlet of said collector, the other of said electrodes being spaced apart from said inlet, preferably at an opposite side of said collector.

Preferably, the other electrode comprises a plurality of electrically connected terminals, said terminals being spaced apart, preferably spaced apart along said opposite side of said collector.

Ideally, said collector comprises a liquid absorbent material, preferably paper.

Ideally, the electrodes are provided on an obverse face and/or a reverse face of said liquid absorbent material, and/or are incorporated into said liquid absorbent material.

Preferably, the collector comprises liquid absorbent material for storing said liquid sample.

Ideally, said liquid absorbent material comprises paper.

According to the present invention, a quantity of liquid-transportable tracer material is provided in said at least one conduit and is exposed in use to said liquid sample when in said at least one conduit.

Ideally, the tracer material is provided on at least one internal surface of said at least one conduit.

Preferably, the tracer material comprises a chemical compound.

Ideally, the tracer material comprises a dye or a fluorescent material.

Preferably, the at least one conduit is shaped and dimensioned to draw said liquid sample into said collector by capillary action.

Ideally, the conduit is substantially straight or curved.

Preferably, a filter is provided between the port and the collector.

Ideally, the filter is provided in the conduit.

Preferably, the filter comprises a microfluidic filter incorporating a plurality of micro fabricated pillars.

Ideally, the conduit comprises a multi-layer formation.

Preferably, the controller includes, or is co-operable with, data storage means and is configured to create a data record of a detected sample in the data storage means.

Ideally, the detection apparatus is configured to generate one or more user alerts upon detection of a sample.

Preferably, the sample detection apparatus comprises an output means configured to transmit information regarding the sample in the collector via wired and/or wireless transmission means. Ideally, the information regarding the sample output from the output means comprises the volume of sample obtained and/or the time it was obtained and/or the date it was obtained.

Preferably, the output means comprises a data transfer port such as a USB or Ethernet or a Bluetooth^{®} or NFC or WiFi or BLE transmitter or an active or passive RFID chip.

Also disclosed is a liquid sample collection apparatus comprising a port for receiving a liquid sample, a collector for storing said liquid sample, at least one conduit connecting said port to said collector, wherein a quantity of liquid-transportable tracer material is provided in said at least one conduit and is exposed in use to said liquid sample when in said at least one conduit.

### Brief Description of the drawings

Embodiments of the invention are now described by way of example and with reference to the accompanying drawings in which like numerals are used to denote like parts and wherein,
Figure 1 shows an exploded view of an embodiment of the liquid sample collection apparatus;
Figure 2 shows a diagram illustrating a conduit incorporated within the liquid sample collection apparatus of Figure 1;
Figure 3 shows a circuit diagram illustrating a sample detection apparatus included in the liquid sample collection apparatus of Figure 1;
Figure 4 shows a diagram illustrating a preferred embodiment of the liquid sample collection apparatus;
Figure 5 is a plan view of the liquid sample collection apparatus of Figure 4 showing the conduit incorporated therein;
Figure 6 shows a plan view of the liquid sample collection apparatus of Figure 4 as well as sectional views A-A and B-B of the port and conduit respectively;

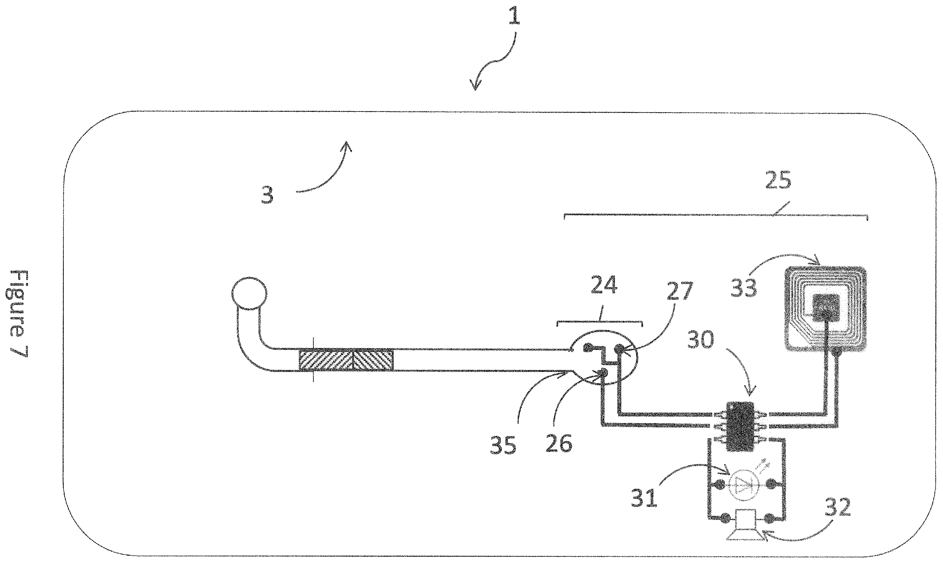

Figure 7 is a schematic diagram of the liquid sample collection apparatus of Figure 4 showing the sample detection apparatus;

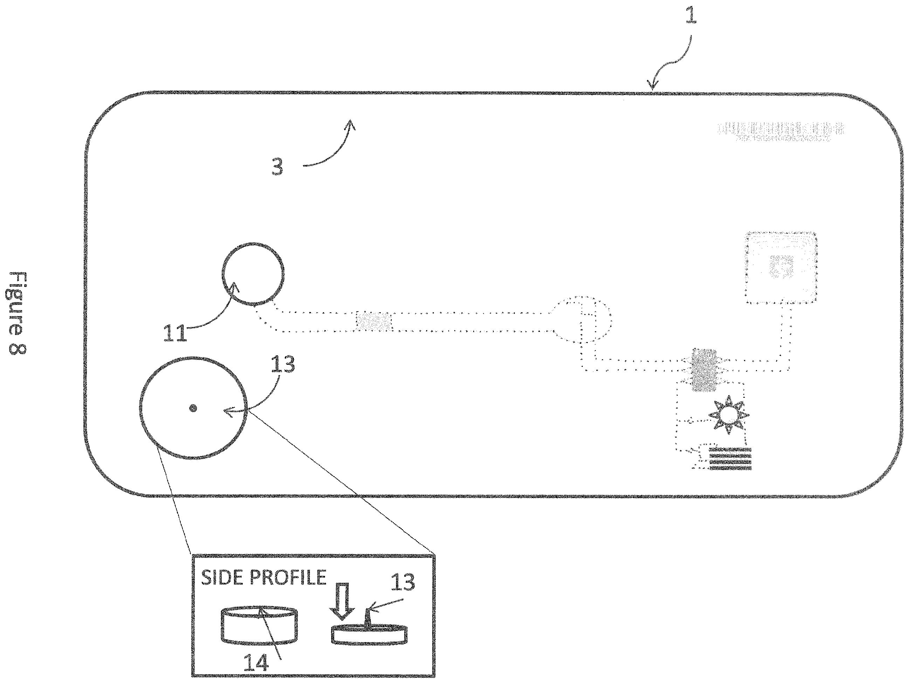

Figure 8 is a plan view of the liquid sample collection apparatus of Figure 4 in particular showing the piercing means provided on the apparatus;

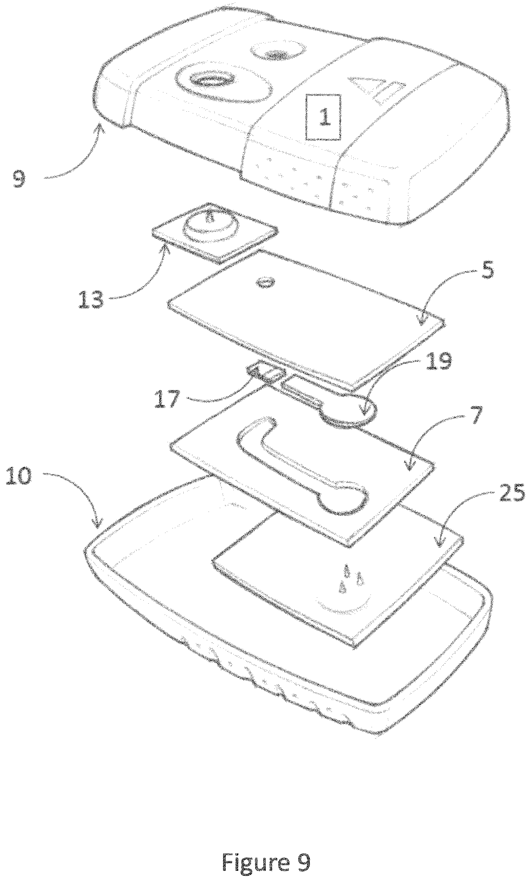

Figure 9 is an exploded view of an alternative preferred embodiment of the liquid sample collection apparatus.

### Detailed Description

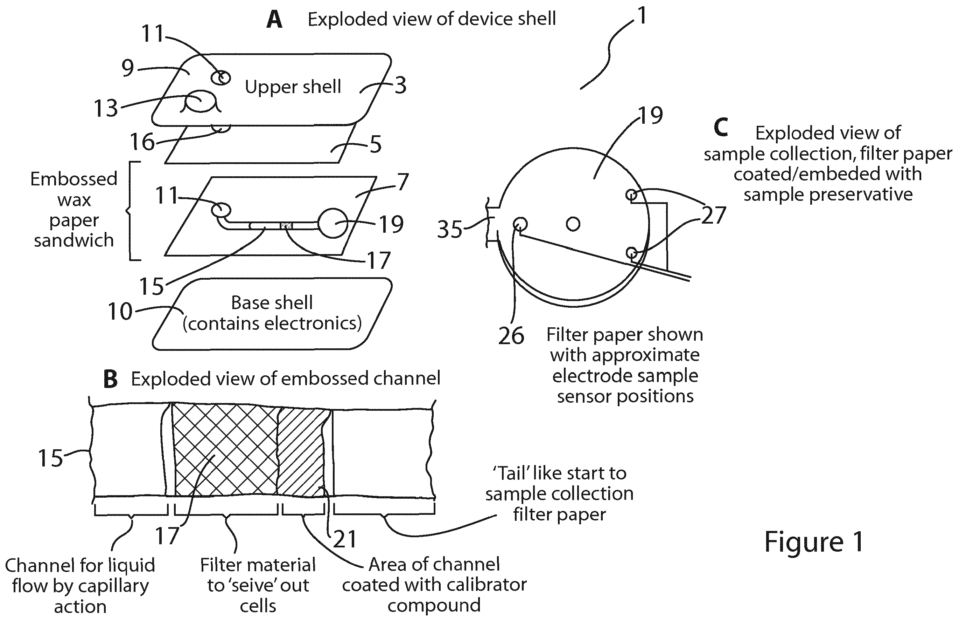

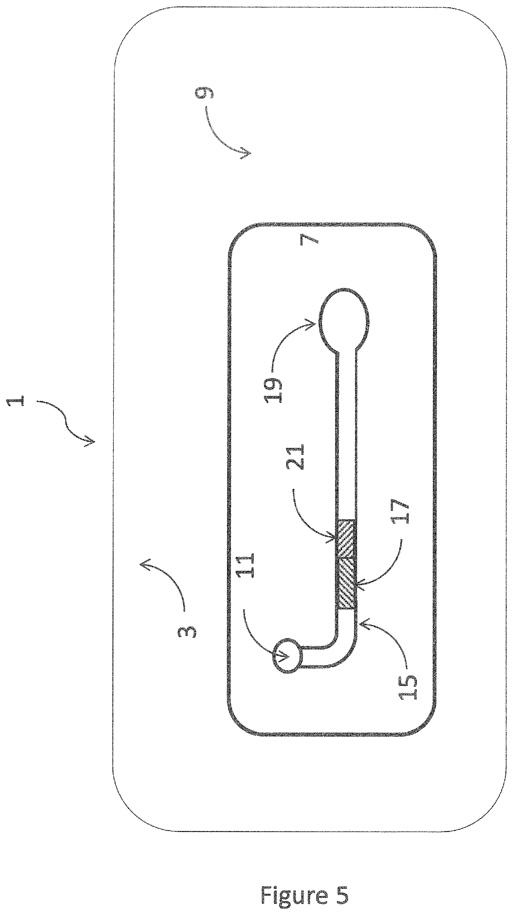

Referring to Figure 1 and 5 of the drawings, there is shown, generally indicated by the reference numeral 1, embodiments of a liquid sample collection apparatus embodying a first aspect of the invention. The apparatus typically includes a housing 3 or other body or support structure, and is preferably portable. The housing 3 typically incorporates a plurality of layers 5, 7. Each layer 5, 7 typically comprises one or more sheets of hydrophobic material. The housing 3 is typically defined by an upper and lower panel 9, 10 with opposing pairs of side, end walls (not illustrated) coupled therebetween, with the side and end walls extending perpendicular relative to each other, such as to define a substantially cuboid shape. The plurality of layers 5, 7 are typically sandwiched between the upper and lower panels 9, 10. Advantageously, the housing 3 provides protection to the contents of the interior, to this end, the housing 3 typically comprises a plastic and/or metal and/or composite and/or any other suitable material.

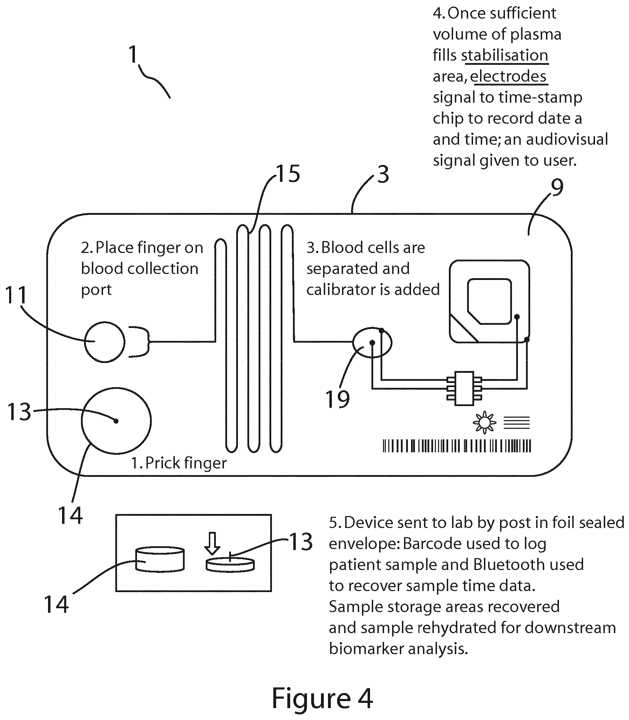

The housing 3 incorporates at least one port 11 for the receipt of a liquid sample. The port 11 may take the form of a receptacle with an aperture, typically formed in the upper panel 9, in which a liquid sample can be deposited. The port 11 typically extends through the upper panel 9 and may also extend through one or more of the plurality of layers 5. The port 11 may incorporate a closing means, e.g. a lid (not shown), which is configured to close the port 11 after the deposit of one or more liquid samples, such as to prevent any unwanted dirt or debris from entering therein which may contaminate the sample. The closing means may comprise any suitable electrical or mechanical device such as a cover variable between an open and closed position, were the variation between the positions may be dependent upon one or more electrical sensors. The port 11 may also be provided on any other suitable panel or wall thereof. The liquid typically comprises a bodily liquid such as blood or urine or saliva however it may also comprise any other suitable liquid. The housing 3 typically incorporates a piercing means 13, as shown in figure 4, for reliably piercing the skin of a user to allow for the passage of a uniform blood droplet from the user. The piercing means may comprise a needle or pin or any other suitable means for piercing a user's skin. Advantageously, the incorporation of the piercing means 13 allows the user to quickly and efficiently deposit a blood sample in the port 11. The piercing means 13 typically incorporates a safety guard 14 to prevent the user accidentally making contact with the piercing means 13, this may comprise a button like arrangement, which when substantially depressed reveals a piercing means 13 therein or alternatively it may comprise a plastic sheath or barrier or any other suitable safety guard. The apparatus 1 may be provided in a vacuum sealed bag and/or the port 11 may be provided with a cover (not shown) such as to ensure the chemical neutrality of the apparatus 1 prior to use.

The housing 3 incorporates a collector 19 for storing the liquid sample as shown in figures 1 and 5. Advantageously, the collector 19 is removable from the apparatus 1 thereby allowing easy analysis of the collected sample. The preferred collector 19 comprises a pad or other structure of liquid absorbent material, preferably paper, however it may also comprise sponge or any other suitable absorbent material. In a preferred embodiment the collector 19 comprises filter paper. The collector 19 may be coated with a stabiliser. The housing 3 may include a chamber for removably receiving the collector 19. The collector 19 is connected or coupled to, or otherwise in liquid communication with, the port 11 via at least one conduit 15. The conduit 15 is shaped and dimensioned to draw the liquid sample from the port 11 to the collector 19 by capillary action, and may for example comprise a micro-fabricated capillary, preferably coated with a calibrating substance. The conduit 15 may be substantially straight or curved or any other suitable shape. The arrangement is such that liquid from the port 11 flows through the conduit 15 and is collected, preferably absorbed, by the collector 19.

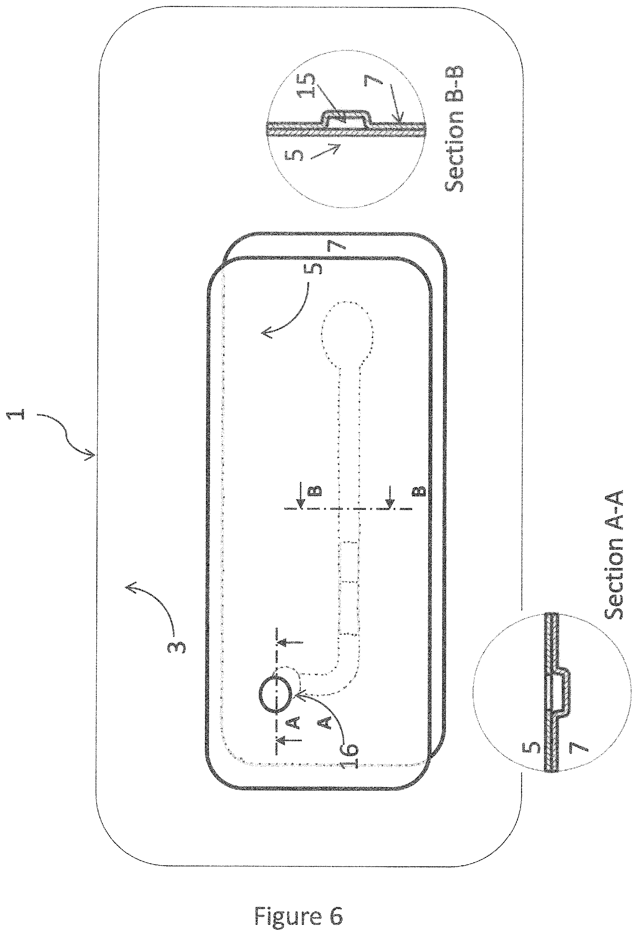

The conduit 15 typically comprises a multi-layer formation as shown in figures 1 and 6. In a preferred embodiment the conduit 15 comprises layers 5, 7 which are typically provided sandwiched between the upper and lower panels 9, 10 of the housing 3. The layers 5, 7 may include complementary formations, e.g. channels, which form the conduit 15 when the layers 5, 7 are brought together. In the embodiment shown there are provided first and second layers 5, 7 which typically comprise a substantially malleable material, such as a bibulous material which has been coated on at least one side in a hydrophobic material. For example, the layers 5, 7 preferably comprise wax paper however they may also comprise any other suitable material. In a preferred embodiment the first and/or second layers 5, 7 are typically embossed using a mould (not illustrated) to define at least one elongate impression (not shown) on an obverse face thereof, the layers 5, 7 are then typically overlaid on top of another such that the at least one elongate impression substantially aligns on each layer 5, 7 whereupon the layers may be coupled together substantially along a peripheral edge of each respective impression to define at least one conduit 15. Typically the conduit(s) extends at least part of the way across the layers 5, 7. In a preferred embodiment the first and second layers 5, 7 are typically coupled together via the application of heat, the wax coating applied to each layer 5,7 binds together when heated. However in alternative embodiments the first and second layers 5, 7 may be coupled via an adhesive or any other suitable material. One of the first or second layers 5, 7 incorporates an aperture 16 for the receipt of the liquid sample into the conduit 15 and to this end the aperture 16 substantially aligns with the port 9 provided on the housing 3 to provide an inlet for the liquid sample.

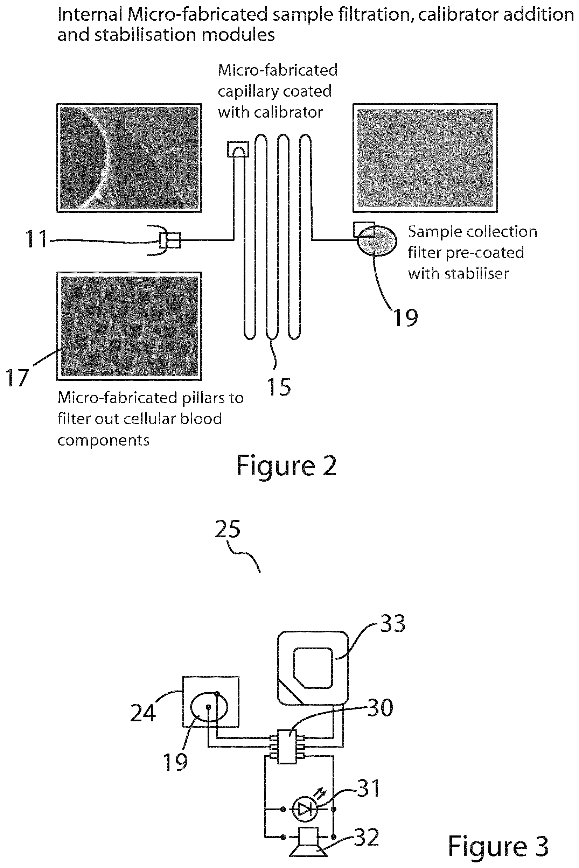

A filter 17 is typically provided between the port 11 and the collector 19. The filter 17 may be of any conventional type that is suitable for isolating/removing/separating one or more components of the liquid sample from other component(s) of the sample. Not only does this allow the desired portion of the sample to reach the collector but it also can help to prevent blockages or coagulation. The filter 17 is typically provided in the conduit 15, for example at or adjacent the port 11. In cases where the liquid sample comprises a blood sample, the filter 17 advantageously separates/removes one or more components from the blood sample such as cells or plasma or any other component. In a preferred embodiment the filter 17 is suitable for filtering out the cells from the blood to leave only the plasma being drawn through the remainder of the conduit 15 to the collection reservoir 19. The filter 17 typically comprises a microfluidic filter incorporating a plurality of micro fabricated pillars as shown in figure 2 which retard and ultimately filter out suspended cells; however it may alternatively comprise a sponge or mesh or gel or glass fibre or any other filter suitable for filtering a liquid.

At least part of the conduit 15 is typically provided with a quantity of liquid-transportable tracer material 21, which is exposed in use to the liquid sample when in the at least one conduit 15. The tracer material 21 is typically coated upon at least one internal surface of the conduit 15, typically after the filter 17 in the direction of liquid flow, thereby ensuring only the desired isolated components of the liquid are coated. The tracer material 21 is taken up by the liquid sample, after having passed through the filter 17, as it flows toward the collector 19. The arrangement may be such that a thin layer chromatography type principle applies whereby only a certain amount of the tracer material 21 reaches the collector 19 carried by the liquid. The tracer material 21 typically comprises a chemical compound (in any form, e.g. solid, powder, liquid, gel that is suitable for being lifted from the conduit surface and carried by the liquid during use) which may for example be coated or sprayed or pipetted onto the internal surface the conduit 15. The tracer material 21 typically comprises a fluorescent material such as fluorescein or a dye such as texas red or cyanine blue. The tracer element 21 allows for the accurate analysis of the liquid sample, as is described in greater detail further on. In a preferred embodiment the tracer element 21 comprises fluorescein, which is detectable under UV light.

The collector 19 is typically coated in, or otherwise provided with, a stabiliser suitable for facilitating protein preservation over a short period of time. Advantageously this allows the apparatus 1 to be used by the user at home and analysed by a medical professional at a later time. The stabiliser may comprise a polar, branched polysaccharide or any other suitable chemical compound. For example, the stabiliser may comprise a dextran, e.g. in the quantity 10mM 70KDa. However alternatively the sample stabiliser may comprise an alginate or other complex polar, branched chain polysaccharide or any other suitable stabiliser. Advantageously as dextran is antithrombotic it also aids the structural stability of any cells contained within the liquid sample.

The liquid sample collection apparatus 1 further comprises a sample detection apparatus 25. The sample detection apparatus 25 typically comprises a sensor 24 for detecting the liquid in the collector 19, and is advantageously configured to generate an output depending on the quantity of the liquid detected in the collector 19. The sample detection apparatus 25 further comprises a controller 30 responsive to the sensor 24 output, which is advantageously configured to determine that the sample has been collected when the sensor 24 indicates that a quantity of the liquid above a threshold amount is detected in the collector 19. The sensor 24 typically comprises an electro conductive sensor which is incorporated within and/or on the collector 19. The sensor 24 (and more particularly the electrodes that are part of the sensor in the preferred embodiment) may be provided on an obverse face and/or a reverse face of the collector 19, and/or incorporated between layers of the collector if the collector comprises a multi-layer structure. The sensor 24 (and in particular the electrodes that are part of the sensor in the preferred embodiment) preferably comprises materials which are inert and which will not compromise the chemical integrity of the sample such as a thin layer of deposit able metal. The sensor 24 is preferably printed on the collector 19, however it may alternatively be coupled via an adhesive or otherwise mounted or coated on the collector.

The sensor 24 typically comprises first and second spaced apart electrodes 26, 27, as shown in figure 1, which are coupled to the collector 19 as indicated above. The sensor output depends on the electrical conductance of the collector 19 between the first and second electrodes 26, 27. The electrical conductance of the collector 19 is dependent on the quantity of liquid collected (i.e. absorbed in preferred embodiments) in use by the collector 19. For example, the sensor 24 may comprise an electrical circuit in which the first and second electrodes 26, 27 serve as a switch, the switch being open or closed depending on the electrical conductance of the collector 19 between the first and second electrodes 26, 27. Typically, the first and second electrodes 26, 27 are mechanically connected to each other by the collector 19 and are selectively electrically connected by the collector 19 depending on the electrical conductance of the collector 19 between the first and second electrodes 26, 27. The first and second electrodes 26, 27 are typically configured to be electrically connected to each other when a quantity of the liquid above a threshold amount is collected by the collector 19. Advantageously the threshold amount corresponds to the desired volume of a collected sample. At least one of said electrodes 26 is typically located adjacent an inlet 35 of the collector 19, the other of the at least one electrodes 27 being spaced apart from the inlet 35, preferably at an opposite side of the collector 19. The other electrode 27 may comprise a plurality of electrically connected terminals. The terminals are typically spaced apart, preferably along the opposite side of the collector 19 from the first electrode 26 as shown in figure 1. Advantageously the plurality of terminals allow for more accurate detection of the sample within the collector 19 as the liquid may not distribute evenly through the collector 19 in-use. The terminals may also be configured to detect when a certain percentage of the sample has been deposited in the collector 19 in-use. In an alternative embodiment the electrodes 26, 27 may be configured to detect the presence of the sample in the collector 19 via measuring the resistivity or induction therein. The electrodes 26, 27 are typically provided on an obverse face and/or a reverse face of the liquid absorbent material of the collector 19, and/or are incorporated into the liquid absorbent material of the collector 19. The electrodes 26, 27 typically incorporate insulation (not illustrated) configured to prevent conductance from any material apart from that of the collector 19.

The controller 30 is responsive to the sensor 24 output and is configured to determine that the sample has been collected when the sensor 24 output indicates that a quantity of the liquid above a threshold amount is detected in the collector 19. In preferred embodiments, the controller 30 includes, or is co-operable with, data storage means (e.g. a memory device) and is configured to create a data record of a valid sample, i.e. a detected sample above the threshold quantity, in the data storage means. The data record may include time stamp information, e.g. the date and/or time at which the sample was detected. For example the controller may include, or be co-operable with a data recording apparatus and trigger an electronic time stamp process in response to the sensor 24 output indicating that the quantity of liquid above the threshold amount has been detected. The sensor 24 is typically electrically coupled to the controller 30. The controller may comprise a microprocessor, or microcontroller or any other suitable controller circuit or circuitry, conveniently in IC form. For example, in use where the liquid sample comprises blood and the collector 19 is collecting blood plasma, the sensor 24 is configured to detect when the desired volume of plasma above the threshold amount has been obtained, the sensor then generates an output to the controller 30 which is configured to activate a data recording apparatus and trigger an electronic time stamp process to record when the plasma sample was collected.

The detection circuit 25 may also be configured to activate one or more user alerts upon activation of the data recording apparatus or otherwise upon detection of a valid sample. The user alerts may be generated by one or more audio, haptic and/or visual alert apparatus 31, 32 as shown in figure 3 (e.g. a lamp, buzzer and/or sounder), activated by the controller upon detection of the sample in the collector 19. Typically these may comprises one or more lights or speakers or any other suitable apparatus, suitable for alerting the user. The audio/visual/haptic alert apparatus 31, 32 are typically electrically coupled to the controller 30. The sample detection apparatus 25 may further comprise an output means 33 configured to transmit information regarding the sample in the collector 19 typically via wired and/or wireless transmission means. The information regarding the sample typically output from the output means 33 may comprise the volume of sample obtained and/or the time it was obtained and/or the date it was obtained or any other relevant information. The output means 33 may comprise a data transfer port such as a USB or Ethernet or any other suitable port suitable for transferring data, the wireless transmission means may comprise a Bluetooth^{®} or NFC or WiFi or BLE transmitter or an active or passive RFID chip or any other suitable wireless transmission means. The data output from the output means 33 may be transmitted to the user's smartphone or any other suitable electronic computing device where upon it may be uploaded to an online database for analysis. The apparatus 1 may have a companion piece of software which is downloadable and/or accessible on computing devices such as to allow a user to regularly monitor their samples and/or notify them if samples have to be obtained at regular intervals. Advantageously this allows the sample to be analysed quickly and reliably on a constant basis. The sample detection apparatus 25 incorporates a power supply (not shown); typically the power supply may comprise a battery such as a watch battery or any other suitable power supply. The sample detection apparatus 25 is typically mounted and/or coupled to the housing 3, preferably upon the interior face of the lower panel 10.

Advantageously, once sufficient volume of the sample has been obtained the user can send the apparatus 1 to a lab for the sample to be assessed by a competent professional. The collector 19, typically comprising filter paper, may be removed from the apparatus 1 and rehydrated to extract biological molecules or drugs. Advantageously, the apparatus 1 is fully disposable so once the collector 19 has been removed for analysis, the remainder of the apparatus 1 may be disposed. The amount of tracer material 21 extracted from the collector 19 can then be related back to the volume of liquid, preferably blood plasma, required to bring the tracer material 21 into the collector 19. Therefore the original volume of plasma which dried within the collector 19 can be calculated.

In an alternative embodiment the apparatus 1 may be modular, typically comprising first and second modules (not shown) which may be coupled together. The first module may comprise the port, collector and conduit and the second module may comprise the sample detection apparatus. Advantageously as the apparatus 1 is typically single use in the embodiments described previously, this modular arrangement would allow for the regular disposal of only the first module which typically comprises low cost components, whilst the second module comprising more expensive components may be used with a plurality of first modules before requiring disposal. This embodiment would also allow a user to collect a plurality of liquid samples over a period of time, for example a week, which could then be analysed on a continual basis.

The apparatus has the potential to be used beyond protein work to include DNA and drug metabolite measurements. It can be applied to many chronic diseases requiring regular monitoring including cancer, diabetes, heart disease and other inflammatory conditions.

The detection circuit 25 could be used where moisture detection is critical to the manufacture of products or in innovative products which require remote monitoring of moisture/ liquids in disposable products such as nappies.

The invention is not limited to the embodiment(s) described herein but can be amended or modified without departing from the scope of the present invention.

## Claims

1. A liquid sample collection apparatus (1) comprising,
a port (11) for receiving a liquid sample,
a collector (19) for storing said liquid sample,
at least one conduit (15) coupling said port (11) to said collector (19), and
a sample detection apparatus (25) comprising
i. a sensor (24) for detecting said liquid in said collector (19) and generating an output depending on the quantity of said liquid detected in said collector (19), and
ii. a controller (30) responsive to said sensor (24) output and configured to determine that said sample has been collected when said sensor (24) output indicates that a quantity of said liquid above a threshold amount is detected in said collector (19);
**characterised in that** a quantity of liquid-transportable tracer material (21) is provided in said at least one conduit (15) and is exposed in use to said liquid sample when in said at least one conduit (15).

2. The apparatus of claim 1, wherein said tracer material (21) is provided on at least one internal surface of said at least one conduit (15).

3. The apparatus of claim 1 or 2, wherein said tracer material (21) comprises a chemical compound.

4. The apparatus of any one of claims 1 to 3, wherein said tracer material (21) comprises a dye or a fluorescent material.

5. The apparatus as claimed in claim 4, wherein said tracer material (21) comprises fluorescein.

## Patentansprüche

1. Vorrichtung zur Entnahme flüssiger Proben (1), umfassend, einen Anschluss (11) zum Aufnehmen einer flüssigen Probe, einen Kollektor (19) zum Aufbewahren der flüssigen Probe, mindestens eine Leitung (15), die den Anschluss (11) mit dem Kollektor (19) koppelt, und
eine Vorrichtung zur Detektion von Proben (25), umfassend
**i.** einen Sensor (24) zum Detektieren der Flüssigkeit in dem Kollektor (19) und zum Erzeugen einer Ausgabe in Abhängigkeit von der Menge der in dem Kollektor (19) detektierten Flüssigkeit, und
ii. eine Steuerung (30), die auf die Ausgabe des Sensors (24) reagiert und dazu konfiguriert ist, zu bestimmen, dass die Probe entnommen wurde, wenn die Ausgabe des Sensors (24) anzeigt, dass eine Menge der Flüssigkeit über einer Schwellenmenge in dem Kollektor (19) detektiert wird;
**dadurch gekennzeichnet, dass** eine Menge an flüssigtransportierbarem Tracer-Material (21) in der mindestens einen Leitung (15) bereitgestellt wird und bei Verwendung der flüssigen Probe ausgesetzt ist, wenn sie sich in der mindestens einen Leitung (15) befindet.

2. Vorrichtung nach Anspruch **1,** wobei das Tracer-Material (21) auf mindestens einer Innenfläche der mindestens einen Leitung (15) bereitgestellt wird.

3. Vorrichtung nach Anspruch 1 oder 2, wobei das Tracer-Material (21) eine chemische Verbindung umfasst.

4. Vorrichtung nach einem der Ansprüche 1-3, wobei das Tracer-Material (21) einen Farbstoff oder ein fluoreszierendes Material umfasst.

5. Vorrichtung nach Anspruch 4, wobei das Tracer-Material (21) Fluorescein umfasst.

## Revendications

1. Appareil de collecte d'échantillon de liquide (1) comprenant,
un port (11) pour recevoir un échantillon de liquide,
un collecteur (19) pour stocker ledit échantillon de liquide, au moins un conduit (15) couplant ledit port (11) audit collecteur (19), et
un appareil de détection d'échantillon (25) comprenant
**i.** un capteur (24) pour détecter ledit liquide dans ledit collecteur (19) et générer une sortie en fonction de la quantité dudit liquide détecté dans ledit collecteur (19), et
ii. un dispositif de commande (30) sensible à la sortie dudit capteur (24) et configuré pour déterminer que ledit échantillon a été collecté lorsque la sortie dudit capteur (24) indique qu'une quantité dudit liquide supérieure à une quantité seuil est détectée dans ledit collecteur (19) ;
**caractérisé en ce qu'**une quantité de matériau traceur transportable par liquide (21) est prévue dans ledit au moins un conduit (15) et est exposée lors de l'utilisation audit échantillon de liquide lorsqu'il se trouve dans ledit au moins un conduit (15).

2. Appareil selon la revendication 1, dans lequel ledit matériau traceur (21) est prévu sur au moins une surface interne dudit au moins un conduit (15).

3. Appareil selon la revendication 1 ou 2, dans lequel ledit matériau traceur (21) comprend un composé chimique.

4. Appareil selon l'une quelconque des revendications 1 à **3,** dans lequel ledit matériau traceur (21) comprend un colorant ou un matériau fluorescent.

5. Appareil selon la revendication 4, dans lequel ledit matériau traceur (21) comprend de la fluorescéine.
